# FASCICULE DE BREVET EUROPEEN

(11) **EP 2 909 308 B1**
(45) Date de publication et mention de la délivrance du brevet: **02.01.2019**
(21) Numéro de dépôt: 13786703.2
(22) Date de dépôt: 11.10.2013
(51) Int. Cl.: C12N 1/20, C12N 1/38

(54) **UTILISATION D'UN COMPOSÉ ANTIOXYDANT POUR LA CULTURE DE BACTÉRIES SENSIBLES À LA TENSION EN OXYGÈNE**
VERWENDUNG EINER ANTIOXIDIERENDEN VERBINDUNG ZUR ZÜCHTUNG VON GEGENÜBER SAUERSTOFFPARTIALDRUCK EMPFINDLICHEN BAKTERIEN
USE OF AN ANTIOXIDANT COMPOUND FOR CULTIVATING BACTERIA SENSITIVE TO OXYGEN TENSION

(30) Priorité: 22.10.2012 FR 1260020; 06.05.2013 FR 1354134
(43) Date de publication de la demande: 26.08.2015
(60) Demande divisionnaire: 18174895.5
(73) Titulaire: Fondation Méditerranée Infection, 13385 Marseille Cedex 05 (FR)
(72) Inventeur: DRANCOURT, Michel, F-13012 Marseille (FR); LA SCOLA, Bernard, F-13790 Chateauneuf Le Rouge (FR); RAOULT, Didier, F-13008 Marseille (FR)
(74) Mandataire: Domange, Maxime
(86) Numéro de dépôt international: PCT/FR2013/052430
(87) Numéro de publication internationale: WO 2014/064359

(56) Documents cités:
- EP-A1- 2 377 919
- US-A- 4 582 807
- N R KRIEG ET AL: "Microaerophily and Oxygen Toxicity", ANNUAL REVIEW OF MICROBIOLOGY, vol. 40, no. 1, 1 octobre 1986 (1986-10-01), pages 107-130, XP055069555, ISSN: 0066-4227, DOI: 10.1146/annurev.mi.40.100186.000543
- A. OMSLAND ET AL: "Host cell-free growth of the Q fever bacterium Coxiella burnetii", PROCEEDINGS OF THE NATIONAL ACADEMY OF SCIENCES, vol. 106, no. 11, 17 mars 2009 (2009-03-17), pages 4430-4434, XP055039511, ISSN: 0027-8424, DOI: 10.1073/pnas.0812074106
- D. A. PODKOPAEVA ET AL: "Oxidative Stress and Antioxidant Cell Protection Systems in the Microaerophilic Bacterium Spirillum winogradskii", MICROBIOLOGY, vol. 72, no. 5, 1 janvier 2003 (2003-01-01), pages 534-541, XP055069026, ISSN: 0026-2617, DOI: 10.1023/A:1026082914661
- B. J. JUVEN ET AL: "Effect of ascorbic, isoascorbic and dehydroascorbic acids on the growth and survival of Campylobacter jejuni", JOURNAL OF APPLIED MICROBIOLOGY, vol. 61, no. 4, 1 octobre 1986 (1986-10-01), pages 339-345, XP055068779, ISSN: 1364-5072, DOI: 10.1111/j.1365-2672.1986.tb04295.x
- Rajiv ET AL: "Effectiveness of Ascorbic Acid as an Oxygen Scavenger in Improving Viability of Probiotic Bacteria in Yoghurts Made with Commercial Starter Cultures", Dairy Journal, vol. 7, no. 435443, 1 January 1997 (1997-01-01), XP055156417,
- I J Kligler ET AL: "THE INFLUENCE OF VITAMIN C ON THE GROWTH OF ANAEROBES IN THE PRESENCE OF AIR, WITH SPECIAL REFERENCE TO THE RELATIVE SIGNIFICANCE OF EH AND 02 IN THE GROWTH OF ANAEROBES", Journal of bacteriology, vol. 35, no. 2, 1 February 1938 (1938-02-01), pages 141-156, XP055362096,
- B P Eddy ET AL: "Interactions between ascorbic acid and bacteria", BACTERIOLOGICAL REVIEWS, vol. 17, no. 2, 1 June 1953 (1953-06-01), pages 93-107, XP055417653, US ISSN: 0005-3678
- Catherine Vilchèze ET AL: "Mycobacterium tuberculosis is extraordinarily sensitive to killing by a vitamin C-induced Fenton reaction", Nature Communications, vol. 4, 21 May 2013 (2013-05-21), page 1881, XP055177685, DOI: 10.1038/ncomms2898

## Description

La présente invention concerne la culture en milieu acellulaire de bactéries dont la croissance est sensible à la tension en oxygène, notamment des bactéries qui tolèrent mal des tensions élevées d'oxygène et pour lesquelles une croissance optimale de la dite bactérie requière une atmosphère d'incubation à teneur en oxygène relativement réduite par rapport à la teneur en oxygène de l'air.

On distingue donc parmi les bactéries sensibles à l'oxygène :
- les bactéries microaérophiles c'est-à-dire qu'elles ne sont pas capables de cultiver sous une atmosphère comprenant la concentration d'oxygène ambiante qui est de environ 21%, notamment entre 1% et 20%, le plus communément à environ 2-2,5%, et
- les bactéries anaérobies strictes c'est-à-dire qu'elles ne sont pas capables de cultiver en présence d'oxygène ou dans des concentrations inférieures aux concentrations de microaérophilie, notamment strictement inférieure à 1%, le plus communément inférieure à 0.1%, idéalement 0%. Pour cultiver les bactéries anaérobies strictes, il faut soit les cultiver dans des étuves ne comportant pas d'oxygène, soit dans des tubes qui ont été désoxygénés et elles ne poussent alors qu'au fond du tube.

Parmi les bactéries anaérobies strictes, on cite plus particulièrement les bactéries extracellulaires, c'est à dire des bactéries qui qui ne peuvent vivre qu'à l'extérieur de cellules.

Parmi les bactéries cultivables en atmosphère microaérophile, on distingue plus particulièrement, les bactéries intracellulaires, mais aussi des bactéries extracellulaires.

On entend ici par «bactérie intracellulaire», une bactérie qui a la capacité de se multiplier au sein d'une cellule hôte. Les bactéries intracellulaires, ayant la faculté de croître dans certaines conditions dans des milieux acellulaires, sont dénommées "bactéries intracellulaires facultatives".

On entend ici par «bactérie intracellulaire facultative », une bactérie qui a la capacité de se multiplier au sein d'une cellule hôte et en milieu acellulaire.

On entend ici par «bactérie extracellulaire, une bactérie qui n'a pas la capacité de se multiplier au sein d'une cellule hôte et se cultive exclusivement en milieu acellulaire.

On entend ici par «milieu de culture acellulaire», un milieu de culture qui ne comprend pas de cellules entières notamment qui ne comprend pas de cellules hôtes entières au sein desquelles la dite bactérie peut se multiplier, lorsque ladite bactérie est intracellulaire ou intracellulaire facultative.

On comprend que les cellules entières doivent être vivantes pour permettre une multiplication de la bactérie en leur sein.

Plus particulièrement, la présente invention concerne la culture de bactéries microaérophiles intracellulaires.

Deux possibilités existent aujourd'hui afin de contrôler la concentration en oxygène dans l'atmosphère utilisée pour l'isolement et la culture des bactéries cultivables en microaérophilie ou en anaérobiose. La première consiste à utiliser des dispositifs de type Gaspak (Oxoid) à base de molécules réductrices qui réagissent et consomment l'oxygène incubé en présence des milieux de culture dans des récipients hermétiques. Un inconvénient de cette approche est qu'elle ne permet pas d'obtenir des concentrations précises en oxygène, ce qui est crucial notamment dans le cas des espèces microaérophiles. La deuxième solution consiste à placer les cultures dans des étuves à concentrations contrôlées en oxygène. Cependant celles-ci sont coûteuses et requièrent une consommation importante en azote ou autre gaz.

Dans l'article Krieg et al. [10], on décrit l'effet de composés oxydants sur la culture de bactéries microaérophiles extracellulaires, notamment Campylobacter jejuni, pour laquelle la cystéine et l'acide ascorbique n'améliorent pas l'aérotolérance (cf. p. 113, 4^{ème} paragraphe :*"cystéine, thioglycolate, mercaptoethanol, thiourea and ascorbic acid do not enhance aerotolerance of C. jejuni on brucella agar and some of these actually inhibit growth...In the case of cysteine... the inhibitory action is likely due to generation of H₂O₂ during autooxidation"*)*.*

L'article Omsland et al. [3] concerne le milieu de culture axénique ACCM contenant de la *L. cystéine.* Il est indiqué que le remplacement de la *L. cystéine* par le glutathion ne permet pas de soutenir la croissance de *Coxiella burnetii* (cf. page 4433, colonne de gauche, fin du 1er paragraphe *:"ACCM, containing the alternative antioxydant L. glutathion instead of L. cysteine, did not support C. burnetii growth").*

L'article de Podkopaeva et al. [11] concerne la culture de la bactérie microaérophile extracellulaire *Spirillum winogradskii* en présence de thiosulfate.

Le brevet US 4,582,807 décrit un milieu de culture pour Mycobactéries comprenant de l'acide ascorbique dans un mélange de vitamines.

L'article de Eddy et Ingram (Bacteriological Reviews, 17(2):93-107, 1953) décrit l'effet de l'acide ascorbique sur les bactéries. Il décrit l'effet protecteur contre l'oxygène pour les bactéries anaérobies et mentionne une étude concernant l'inhibition de Mycobacterium par l'acide ascorbique.

Le but de la présente invention est d'améliorer et faciliter les conditions de croissance en culture acellulaire des bactéries dont la croissance est sensible à la tension en oxygène et notamment des bactéries qui tolèrent mal des tensions élevées d'oxygène et pour lesquelles une croissance optimale de la dite bactérie requière une atmosphère d'incubation à teneur en oxygène relativement réduite par rapport à la teneur en oxygène de l'air, lesdites bactéries étant choisies parmi les bactéries les bactéries microaérophiles intracellulaires du type mycobactéries et notamment *Mycobacterium tuberculosis.*

On entend ici par «atmosphère microaérophile», de l'air appauvri en oxygène avec une proportion molaire en oxygène inférieure à 10%, de préférence 5%, de préférence encore inférieure à 2,5%. Pour les bactéries anaérobies strictes, la teneur en oxygène doit être proche de 0%, notamment inférieure à 0.1%, comme mentionné ci-dessus, la tolérance à de très faibles quantités d'oxygène étant variable selon les espèces de bactéries anaérobies.

Les inventeurs ont découvert de façon fortuite que l'ajout de certains composés antioxydants dans un milieu de culture acellulaire pouvait permettre de:
- améliorer la croissance des dites bactéries en obtenant plus rapidement des bactéries en concentration suffisante pour être détectables après multiplication et/ou en augmentant la concentration en bactéries après un délai donné de culture, c'est-à-dire par unité de temps, et
- cultiver avec au moins un même niveau de croissance, dans une atmosphère contenant des tensions d'oxygène plus élevées, desdites bactéries intracellulaires microaérophiles, cultivables en l'absence de composé antioxydant, en atmosphère microaérophile, voire avec un niveau de croissance plus élevé ; et
- cultiver avec un niveau de croissance plus élevé, en atmosphère microaérophile, des bactéries cultivables, en l'absence de composé antioxydant, dans une atmosphère contenant une tension d'oxygène plus élevée, mais inférieure à la teneur en oxygène de l'air, comme c'est le cas pour la bactérie intracellulaire facultative *Mycobacterium tuberculosis.*

La présente invention fournit donc un procédé de culture in vitro de bactéries en milieu de culture acellulaire, de bactéries dont la croissance est sensible à la teneur en oxygène, la croissance optimale desdites bactéries exigeant une atmosphère d'incubation à teneur en oxygène relativement réduite, voire nulle, par rapport à la teneur en oxygène de l'air, lesdites bactéries étant choisies parmi les bactéries microaérophiles intracellulaires du type des mycobactéries, caractérisé en ce que l'on ajoute dans le dit milieu de culture acellulaire au moins un un composé antioxydant l'acide ascorbique, à une concentration d'au moins 100 µg/ml, et l'on cultive la dite bactérie dans ledit milieu de culture en présence d'oxygène.

Plus particulièrement, les inventeurs ont découvert que l'ajout d'un composé antioxydant confère un effet tampon au regard de la teneur en oxygène pour les bactéries, notamment les bactéries anaérobies strictes et les bactéries intracellulaires car cet ajout permet de tolérer une croissance en présence de teneur en oxygène relativement plus élevée, notamment pour les bactéries microaérophiles intracellulaires. Et, cet ajout permet aussi pour certaines bactéries telles que *Mycobacterium tuberculosis* cultivables à des tensions d'oxygènes plus élevées en l'absence de composé antioxydant, d'améliorer leur croissance à des tensions en oxygène diminuées en présence de composé antioxydant.

Les inventeurs formulent l'hypothèse non encore totalement élucidée et démontrée que l'effet du composé antioxydant pourrait provenir d'une réaction avec les radicaux libres oxygénés toxiques ayant un effet inhibiteur de la toxicité des dits radicaux à l'encontre de la croissance de la dite bactérie. Ces radicaux résultant de l'action de l'oxygène sur des substances issues des bactéries et/ou milieu de culture, seraient responsables des difficultés de culture et mauvaise tolérance de la croissance des dites bactéries en présence de teneurs élevées d'oxygène.

Plus particulièrement, le dit milieu acellulaire est un milieu autre qu'un milieu constitué essentiellement à partir d'un lysat ou broyat cellulaire d'un type de cellules eucaryotes donné, notamment autre qu'un filtrat de dit broyat ou lysat, de préférence un milieu autre qu'un milieu acellulaire de lysat ou broyat cellulaire d'un type de cellules eucaryotes donné au sein desquelles la dite bactérie peut être cultivée, notamment autre qu'un filtrat de dit broyat ou lysat, notamment lorsqu'il s'agit d'une bactérie intracellulaire.

Plus particulièrement, le dit milieu acellulaire est choisi parmi un milieu axénique constitué de substances chimiques ou biologiques défini qualitativement et quantitativement, et un milieu comprenant un extrait de broyat ou lysat de tissu pluricellulaire.

Plus particulièrement encore, le dit milieu acellulaire est un milieu axénique constitué de substances chimiques ou biologiques défini qualitativement et quantitativement, lorsque la dite bactérie est une bactérie intracellulaire facultative, et le dit milieu acellulaire est constitué à partir d'un broyat ou lysat de tissu pluricellulaire, notamment un filtrat de dit broyat ou lysat, notamment de tissu sanguin ou tissu de coeur et/ou poumon, lorsque ladite bactérie est une bactérie extracellulaire.

De tels milieux de culture acellulaires qu'ils soient liquides, solides ou biphasiques sont bien connus de l'homme de l'art.

Plus particulièrement, on cultive la dite bactérie dans une dite atmosphère d'incubation comprenant une proportion molaire en oxygène supérieure à la tension maximale tolérée en l'absence de composé antioxydant pour un même niveau de croissance dans une même durée de culture.

En pratique, plus particulièrement encore, on cultive la dite bactérie dans une dite atmosphère d'incubation comprenant une proportion molaire en oxygène inférieure ou égale à 20%.

Avantageusement toutefois, on cultive lesdites bactéries selon la présente invention dans une atmosphère comprenant une teneur en oxygène supérieure à 5%, notamment dans de l'air contenant 5% de CO₂ (soit une teneur en oxygène inférieure à 16%), voire dans une atmosphère aérobie.

Plus particulièrement encore, dans certains cas, comme explicité ci-après, on cultive la dite bactérie microaérophile intracellulaire dans une dite atmosphère d'incubation microaérophile comprenant une proportion molaire en oxygène inférieure à 5%, de préférence de 2 à 5%, de préférence encore 2,5%.

De préférence, on ajoute dans ledit milieu de culture une concentration déterminée d'un composé antioxydant déterminé.

Les inventeurs ont en effet testé différentes molécules présentant une activité antioxydante et ont découvert que certains composés anti-oxydants dans certaines concentrations présentent un effet supérieur sur la croissance des dites bactéries.

Plus particulièrement, le dit milieu de culture comprend au moins un autre composé antioxydant choisi parmi le glutathion (y-L-Glutamyl-L-cysteinylglycine) et l'hydrosulfure de sodium. L'acide ascorbique et le glutathion sont préférés car ils sont capables à des doses précises de permettre la culture à un niveau d'oxygène plus élevé.

Plus particulièrement encore, le dit composé antioxydant est mis en oeuvre à une concentration de 100 µg/ml à 2 mg/ml, ou concentration molaire de 10⁻⁵ M à 10⁻² M.

Plus particulièrement, la dite bactérie est une bactérie cultivable dans un dit milieu de culture en l'absence de dit composé oxydant sous une atmosphère d'incubation comprenant une proportion molaire en oxygène inférieure à la proportion molaire d'oxygène dans l'air, de préférence inférieure à 20%, et on cultive la dite bactérie en présence de dit composé oxydant dans le dit milieu de culture sous une atmosphère d'incubation comprenant une teneur en oxygène inférieure ou égale à la proportion d'oxygène dans l'air, de préférence inférieure à 20%, de préférence encore supérieure à 5%.

Dans un mode de réalisation, la dite bactérie est cultivée dans un dit milieu de culture acellulaire, sous atmosphère d'incubation microaérophile avec une proportion molaire en oxygène de pas plus de 5%, de préférence pas plus de 2,5% dans l'atmosphère d'incubation, en l'absence de dit composé antioxydant, et on cultive la dite bactérie en présence de dit composé antioxydant dans un dit milieu de culture sous une atmosphère d'incubation microaérophile comprenant une proportion molaire en oxygène entre 2,5% et 20%, de préférence entre 5% et 16%, notamment de l'air enrichi à 5% de CO₂.

Plus particulièrement les mycobactéries sont choisies parmi, de préférence, *Mycobacterium tuberculosis, Mycobacterium leprae* et *Mycobacterium ulcerans.*

Les bactéries intracellulaires vivent avec une tension d'oxygène qui est celle à l'intérieur des cellules qui est de 5%, qui est beaucoup plus basse que celle de l'air ambiant (environ 21%). La croissance des mycobactéries en milieu acellulaire, notamment *Mycobacterium tuberculosis* requière une atmosphère d'incubation à teneur en oxygène réduite par rapport à la teneur de l'air, notamment de l'air enrichi à 5% de CO₂. Cependant les inventeurs ont mis en évidence que leur difficulté de croissance dans un tel milieu était due à une mauvaise tolérance à l'oxygène et ils ont mis en évidence que les bactéries du complexe tuberculeux *Mycobacterium tuberculosis,* en présence de composé antioxydant, poussaient mieux à une tension réduite d'oxygène de l'ordre de 5% que quand elles étaient exposées à de l'air enrichi à 5% de CO₂. Les inventeurs ont donc découvert que, en présence de composé antioxydant, il est possible d'améliorer la croissance dans de l'air enrichi à 5% de CO₂ et donc de se passer de cultiver ces bactéries dans une tension davantage diminuée en oxygène. Et, de manière tout à fait surprenante, selon la présente invention, les bactéries ont une meilleure croissance, non seulement en tension normale en oxygène dans de l'air à 5% de CO₂, mais encore meilleure en tension contrôlée en oxygène à moins de 5% d'oxygène. Ceci signifie que le composé antioxydant constitue d'une certaine manière un facteur de croissance pour les mycobactéries même lorsqu'elles sont cultivées lors de basse tension d'oxygène.

Plus particulièrement encore, la dite bactérie est *Mycobacterium tuberculosis.*

Plus particulièrement encore, le dit milieu de culture axénique est le milieu dénommé ACCM [3] comprenant les composant suivants aux concentrations suivantes: tampon citrate (acide citrique: 2,5 g/ml; citrate de sodium tribasique: 4,74 mg/ml); du phosphate de potassium (0,5 mg/ml), du chlorure de magnésium (0,2 mg/ml), du chlorure de calcium (0,013 mg/ml), du sulfate de fer (2 µg/ml), de la L-cystéine (0,26 mg/ml), de la néopeptone (0,1 mg/ml), des casamino acides (2,5 mg/ml), de la méthyl béta cyclodextrine (1 mg/ml). Ce milieu de culture est un milieu chimiquement défini décrit dans WO 2010/096640 dénommé ACCM (ACCM=acidified citrate cystéine medium) amélioré en AACM-2 adapté aux bactéries pathogènes donnant comme exemple C. burnetii. Ce milieu a permis l'obtention de colonies (0,01 mm de diamètre) en 6 jours.

Selon la présente invention, en présence de composé antioxydant dans un dit milieu axénique, sous une atmosphère à proportion molaire en oxygène inférieure à 20%, notamment inférieure à 16% (de l'air enrichi à 5% de CO₂), on peut multiplier la concentration de bactérie Coxiella burnetii d'au moins un facteur 10 (1 log) en pas plus de 9 jours.

Pour l'isolement et la culture des mycobactéries du complexe *Mycobacterium tuberculosis,* on dispose de milieux solides, de milieux liquides, et de milieux biphasiques comportant une phase liquide et une phase solide. Les milieux solides sont fabriqués à base de gélose ou d'agar. Les milieux contenant de l'oeuf entier sont d'utilisation très courante et le milieu le plus utilisé est le milieu de Lowenstein Jensen.

Une catégorie de milieux solides sont les milieux à l'agar en particulier le milieu Middlebrook 7H10 et le milieu 7H11 (milieu 7H10 plus 0,1% de caséine hydrolysée). Le milieu de Middlebrook contient 2% de glycérol, qui facilite la culture des mycobactéries du complexe Mycobacterium avium. Les milieux liquides correspondent essentiellement au milieu Middlebrook 7H9.

Cependant, l'isolement des mycobactéries du complexe *Mycobacterium tuberculosis* est lent puisque la totalité des souches des différentes espèces du complexe *Mycobacterium tuberculosis* est isolée dans un délai compris entre 6 et 8 semaines. Tout gain de temps sur ce délai représente donc une amélioration significative du diagnostic de laboratoire de la tuberculose et les autres infections à mycobactérie.

Dans un mode de réalisation plus particulier, la dite bactérie est une bactérie *Mycobacterium tuberculosis,* le dit composé anti-oxydant étant l'acide ascorbique à une concentration d'au moins 100 µg/ml, sous une atmosphère d'incubation microaérophile contenant une proportion molaire en oxygène inférieure à 20%, de préférence encore de 2 à 5%, le dit milieu de culture étant de préférence un milieu de type Middlebrook.

Plus particulièrement encore, ledit milieu de culture de *Mycobacterium tuberculosis* est un milieu de culture solide type Middlebrook de référence 7H10, additionné de produit gélifiant choisi de préférence parmi les géloses et agar, de préférence dans une proportion pondérale de 0.5 à 5%, de préférence encore de 1 à 2%.

Ainsi, selon la présente invention, on peut multiplier la concentration de bactérie *Mycobacterium tuberculosis* d'au moins un facteur 10 (1 log), en pas plus de 5 jours sous atmosphère à proportion molaire en oxygène inférieure à 20%, notamment inférieure à 16% (de l'air enrichi à 5% de CO₂), et en pas plus de 48 heures en microaérophilie avec une proportion molaire d'oxygène inférieure à 5%. On peut même détecter par autofluorescence des minicolonies de bactéries *Mycobacterium tuberculosis* de 0,3 mm en 36 heures. De tels niveaux de croissance dans un temps aussi court n'ont jamais été rapportés dans la littérature.

On a décrit dans WO 2010063911 un milieu de culture permettant l'isolement plus rapide des mycobactéries du complexe *Mycobacterium tuberculosis* et des autres mycobactéries, à partir d'échantillons de prélèvement cliniques. Ce milieu de culture permet l'isolement des mycobactéries, aussi bien en milieu liquide adapté aux automates de détection qu'en milieu solide pour une détection manuelle, et, de surcroît, est compatible avec une décontamination à la chlorhexidine dans le cas de prélèvements cliniques pouvant comprendre des bactéries de la flore commensale risquant d'inhiber la croissance des mycobactéries. Ce milieu de culture de mycobactéries, comprend des facteurs de croissance de mycobactéries et, de préférence, des antibiotiques sans activité à l'égard des mycobactéries, et il est caractérisé en ce qu'il comprend les composants additionnels suivants :
- de la lécithine, et
- du sang défibriné, et
- du sérum de veau foetal décomplémenté.

Le sérum de veau foetal est décomplémenté, c'est-à-dire que l'on lui a retiré, de façon connue, l'ensemble des protéines appelées "complément sérique" par chauffage, notamment à 56°C pendant une heure. Ce complément sérique est connu pour avoir une activité antibactérienne.

Ce milieu de culture de mycobactéries est constitué d'un milieu de culture de base, connu pour la culture des mycobactéries, comprenant notamment des sels minéraux, sucres, acides aminés, protéines et vitamines, ledit milieu de culture de base étant supplémenté par lesdits composants additionnels mentionnés ci-dessus.

Selon des caractéristiques préférées de réalisation :
- la lécithine est comprise dans une proportion pondérale de 0,1 à 5%, de préférence 0,5 à 1%,
- la lécithine est de la lécithine de jaune d'oeuf,
- le sérum de veau foetal décomplémenté est compris dans une proportion en volume de 2,5 à 25%, de préférence de 10 à 20%,
- le sang est compris dans une proportion en volume de 2,5 à 15%, de préférence de 5 à 10%,
- le sang est du sang défibriné de lapin ou, de préférence, de mouton.

Plus particulièrement, un milieu de culture de mycobactéries selon l'invention comprend un milieu de culture de mycobactéries de base comprenant, outre de l'eau distillée, les composants suivants : sulfate d'ammonium, sulfate de magnésium, sulfate de cuivre, sulfate de zinc, citrate de sodium, citrate d'ammonium ferrique, chlorure de sodium, chlorure de calcium, phosphate monopotassique, phosphate disodique, acide L-glutamique, biotine et pyridoxine. Ces composants sont, notamment, les composants contenus dans les milieux de culture de mycobactéries dénommés milieux Middlebrook.

En pratique, ce milieu de culture se présente initialement sous forme d'un liophylisat desdits composants listés ci-dessus, destiné à être dilué dans de l'eau distillée pour former le milieu de culture selon l'invention.

Selon une première variante de réalisation de l'invention, ledit milieu de culture de *Mycobacterium tuberculosis* est un milieu de culture liquide.

Plus particulièrement, un milieu de culture de mycobactéries selon l'invention comprend un milieu de culture de base qui est un milieu liquide Middlebrook de référence 7H9 et des facteurs de croissance de mycobactéries additionnels suivants : hydrolysat de caséine, glycérol, polysorbate, hemin, acide lactique, biotine, stéarate de polyoxyéthylène, sérum albumine bovine, dextrose et de préférence du supplément H. Le supplément H est une digestion de caséine hautement raffinée.

Selon une seconde variante de réalisation d'un milieu de culture selon l'invention, ledit milieu de culture est un milieu de culture aqueux solide de type Middlebrook de référence 7H10, additionné de dit produit gélifiant et des facteurs de croissance de mycobactéries additionnels suivants : acide oléique, albumine bovine, de préférence la fraction V de l'albumine bovine, dextrose, catalase et glycérol. Le vert de malachite, contenu dans le milieu 7H10, est un inhibiteur de croissance des bactéries autres que mycobactéries. Les facteurs de croissance de mycobactéries additionnels ci-dessus, autres que le glycérol, sont connus sous la dénomination facteurs OADC.

D'autres caractéristiques et avantages de l'invention sont présentés dans la description détaillée d'un exemple de réalisation .

### Exemple: Effet de l'acide ascorbique sur la croissance axénique de Mycobacterium tuberculosis

Les inventeurs ont testé l'effet de l'acide ascorbique à la concentration finale de 100 µg/mL sur la croissance de *Mycobacterium tuberculosis* en milieu axénique. Pour ce faire, les inventeurs ont utilisé 3 souches comprenant la souche de référence *Mycobacterium tuberculosis* H37Rv et deux souches cliniques issues de l'activité de routine diagnostique du laboratoire. Ces deux souches ont été isolées de prélèvement d'expectoration de deux patients différents. Ces souches ont été calibrées à 10⁶ mycobactéries/mL dans du tampon phosphate puis ont été déposées sur une gélose Middlebrook 7H10 en présence ou non d'acide ascorbique à la concentration finale de 100 µg/mL. Les géloses ont été incubées sous une atmosphère anaérobie stricte (pas d'oxygène) obtenue par technique Gazpack (Oxoid), et en atmosphère microaérophile (2,5% d'O₂) obtenue par technique Gazpack ou encore en air enrichi en CO₂ contenant 5% de CO₂ dans un incubateur à 37°C. Les inventeurs ont suivi le développement des colonies bactériennes par l'observation des colonies en autofluorescence [1, 2] (méthode qui n'est pas utilisée en routine pour la détection de microcolonies de mycobactéries) et l'identification a été confirmée après coloration de Ziehl-Neelsen par analyse en spectrométrie de masse [6].

Ces travaux ont montré une détection de colonies de *Mycobacterium tuberculosis* pour les trois souches, dès 36 heures d'incubation en présence d'acide ascorbique (100 µg/mL) mais pas de colonies à 36 h en absence d'acide ascorbique, en atmosphère de microaérophilie.

Une croissance améliorée de 1 log bactéries par rapport au témoin cultivé sans antioxydant avec une atmosphère microaérophile à 2,5%, a été observé à 48 heures, en présence d'acide ascorbique (100 µg/mL) (5,6 x 10⁻⁴ M) et également un accroissement de 1 log par rapport au témoin positif en 5 jours en atmosphère à 5% CO₂. Des minicolonies de 0,3 mm ont été détectées en autofluorescence dès 36h. L'autofluorescence des mycobactéries est connue [1, 2]. Le nombre de colonies détectées à 48hr était plus important en présence qu'en l'absence de composé antioxydant dans tous les cas, et le nombre de colonies détectées à 48hr était plus important, en présence de composé antioxydant, en atmosphère microaérophile que dans l'air enrichi à 5% de CO₂.

### Références bibliographiques

[1] Joshi P, Singh M, Bhargava A, Singh M, Mehrotra R. Autofluorescence-an important ancillary technique for the detection of Mycobacterium tuberculosis: Revisited. Diagn Cytopathol. 2012 Feb 20. doi: 10.1002/dc.21860. [Epub ahead of print].
[2] Patiño S, Alamo L, Cimino M, Casart Y, Bartoli F, Garcia MJ, Salazar L. Autofluorescence of mycobacteria as a tool for detection of Mycobacterium tuberculosis. J Clin Microbiol. 2008;46:3296-302.
[3] Omsland A, Cockrell DC, Howe D, et al. Host cell-free growth of the Q fever bacterium Coxiella burnetii. Proc. Natl. Acad. Sci. U.S.A. 2009; 106:4430-4434.
[4] Gimenez DF. STAINING RICKETTSIAE IN YOLK-SAC CULTURES72. Stain Technol 1964 May;39:135-40.
[5] Angelakis E, Richet H, Rolain J-M, La Scola B, Raoult D. Comparison of real-time quantitative PCR and culture for the diagnosis of emerging Rickettsioses. PLoS Negl Trop Dis 2012; 6:e1540.
[6] El Khéchine A, Couderc C, Flaudrops C, Raoult D, Drancourt M. Matrix-assisted laser desorption/ionization time-of-flight mass spectrometry identification of mycobacteria in routine clinical practice. PLoS One. 2011;6(9):e24720).
[7] Fenner L, Roux V, Mallet MN, Raoult D. Bacteroides massiliensis sp. nov., isolated from blood culture of a newborn. Int J Syst Evol Microbiol. 2005 May;55:1335-7.
[8] Fenollar F, Fournier PE, Raoult D. Molecular detection of Coxiella burnetii in the sera of patients with Q fever endocarditis or vascular infection. J Clin Microbiol 2004; 42:4919-24).
[9] La Scola B, Fournier PE, Raoult D. Burden of emerging anaerobes in the MALDI-TOF and 16S rRNA gene sequencing era. Anaerobe 2011 ; 17:106-112
[10] N R Krieg et al. Microaerophily and oxygen toxicity. Annual review of microbiology, vol. 40, n°1, 1986, pages 107-130
[11] D. A. Podkopaeva et al. Oxidative stress and antioxidant cell protection systems in the microaerophilic bacterium Spirillum winogradskii. Microbiology, vol. 72, n°5, 2003, pages 534-541

## Revendications

1. Procédé de culture in vitro en milieu de culture acellulaire, de bactérie dont la croissance est sensible à la teneur en oxygène, la croissance optimale de la dite bactérie exigeant une atmosphère d'incubation à teneur en oxygène relativement réduite par rapport à la teneur en oxygène de l'air, ladite bactérie étant choisie parmi les bactéries microaérophiles intracellulaires du type mycobactéries, **caractérisé en ce que** l'on ajoute dans le dit milieu de culture acellulaire au moins un composé antioxydant, l'acide ascorbique à une concentration d'au moins 100 µg/ml et l'on cultive la dite bactérie dans ledit milieu de culture en présence d'oxygène.

2. Procédé selon la revendication 1, **caractérisé en ce que** le dit milieu de culture comprend en outre un composé antioxydant choisi parmi le glutathion et l'hydrosulfure de sodium.

3. Procédé selon la revendication 1 ou 2, **caractérisé en ce que** le dit milieu acellulaire est choisi parmi :
- un milieu axénique constitué de substances chimiques ou biologiques défini qualitativement et quantitativement, et
- un milieu comprenant un extrait de broyat ou lysat de tissu pluricellulaire.

4. Procédé selon l'une des revendications 1 à 3, **caractérisé en ce que** l'on cultive la dite bactérie dans une dite atmosphère d'incubation comprenant une proportion molaire en oxygène supérieure à la tension maximale tolérée en l'absence de composé antioxydant pour un même niveau de croissance dans une même durée de culture.

5. Procédé selon l'une des revendications 1 à 4, **caractérisé en ce que** le dit composé antioxydant est mis en oeuvre à une concentration de 100µg/ml à 2 mg/ml.

6. Procédé de culture selon l'une des revendications 1 à 5, **caractérisé en ce que** la dite bactérie est une dite bactérie microaérophile intracellulaire du type mycobactéries apte à être cultivée dans un dit milieu de culture acellulaire, sous atmosphère d'incubation microaérophile avec une proportion molaire en oxygène de pas plus de 2.5% dans l'atmosphère d'incubation, en l'absence de dit composé antioxydant, et on cultive la dite bactérie en présence de dit composé antioxydant dans un dit milieu de culture sous une atmosphère d'incubation comprenant une proportion molaire en oxygène entre 2,5% et 20%.

7. Procédé selon l'une des revendications là 6, **caractérisé en ce que** la dite bactérie est choisie parmi les bactéries *Mycobacterium tuberculosis; Mycobacterium leprae* et *Mycobacterium ulcerans.*

8. Procédé selon la revendication 7, **caractérisé en ce que** la dite bactérie est une bactérie *Mycobacterium tuberculosis.*

9. Procédé selon la revendication 8 **caractérisé en ce qu'**on réalise une culture sous une atmosphère d'incubation microaérophile contenant une proportion molaire en oxygène inférieure à 20%.

10. Procédé selon l'une des revendications 8 à 9 **caractérisé en ce que** le dit milieu de culture est un milieu de type Middlebrook.

11. Procédé selon l'une des revendications 8 à 10 **caractérisé en ce que** la dite bactérie *Mycobacterium tuberculosis* est cultivée en présence de composé antioxydant sous une atmosphère d'incubation microaérophile qui contient une proportion molaire en oxygène inférieure à 5 %.

12. Procédé selon l'une des revendications 8 à 10 **caractérisé en ce que** la dite bactérie *Mycobacterium tuberculosis* est cultivée en présence de composé antioxydant sous une atmosphère d'incubation microaérophile qui est de l'air enrichi en CO₂ à 5 %.

13. Procédé selon la revendication 10 **caractérisé en ce que** ledit milieu de culture est un milieu de culture solide à base de gélose ou agar, de préférence avec 1 à 2% d'agar.

14. Procédé selon la revendication 10 **caractérisé en ce que** ledit milieu de culture comprend des facteurs de croissance de mycobactéries additionnels choisis parmi les acide oléique, albumine bovine, fraction V de l'albumine bovine, dextrose, catalase et glycérol.

15. Procédé selon l'une des revendications 1 à 12 **caractérisé en ce que** ledit milieu est un milieu de culture solide.

## Patentansprüche

1. Verfahren zur In-vitro-Kultur in einem azellulären Kulturmedium eines Bakteriums, dessen Wachstum gegenüber dem Sauerstoffgehalt empfindlich ist, wobei das optimale Wachstum des Bakteriums eine Inkubationsatmosphäre mit einem im Vergleich zum Sauerstoffgehalt der Luft relativ niedrigen Sauerstoffgehalt erfordert, wobei das Bakterium ausgewählt ist aus intrazellulären mikroaerophilen Bakterien vom Mykobakterientyp, **dadurch gekennzeichnet, dass** mindestens eine antioxidative Verbindung, Ascorbinsäure, dem azellulären Kulturmedium in einer Konzentration von mindestens 100 µg/ml zugesetzt wird und das Bakterium in dem Kulturmedium in Gegenwart von Sauerstoff gezüchtet wird.

2. Verfahren nach Anspruch 1, **dadurch gekennzeichnet, dass** das Kulturmedium ferner eine antioxidative Verbindung umfasst, ausgewählt aus Glutathion und Natriumhydrosulfid.

3. Verfahren nach Anspruch 1 oder 2, **dadurch gekennzeichnet, dass** das azelluläre Medium ausgewählt ist aus:
- einem axenischen Medium, das aus chemischen oder biologischen Substanzen besteht, die qualitativ und quantitativ definiert sind, und
- einem Medium, das ein Extrakt aus zerkleinertem oder lysiertem multizellulärem Gewebe umfasst.

4. Verfahren nach einem der Ansprüche 1 bis 3, **dadurch gekennzeichnet, dass** das Bakterium in einer Inkubationsatmosphäre kultiviert wird, die einen molaren Anteil an Sauerstoff umfasst, der höher als der maximale Druck ist, der bei Abwesenheit einer antioxidativen Verbindung für das gleiche Wachstumsniveau in der gleichen Kulturzeit toleriert wird.

5. Verfahren nach einem der Ansprüche 1 bis 4, **dadurch gekennzeichnet, dass** die antioxidative Verbindung in einer Konzentration von 100 µg/ml bis 2 mg/ml verwendet wird.

6. Kulturverfahren nach einem der Ansprüche 1 bis 5, **dadurch gekennzeichnet, dass** es sich bei dem Bakterium um ein intrazelluläres mikroaerophiles Bakterium vom Mykobakterientyp handelt, das geeignet ist für die Züchtung in dem azellulären Kulturmedium unter mikroaerophiler Inkubationsatmosphäre mit einem molaren Sauerstoffanteil von nicht mehr als 2,5 % in der Inkubationsatmosphäre in Abwesenheit der antioxidativen Verbindung, wobei das Bakterium in Gegenwart der antioxidativen Verbindung in dem Kulturmedium unter einer Inkubationsatmosphäre kultiviert wird, die einen molaren Sauerstoffanteil zwischen 2,5% und 20% umfasst.

7. Verfahren nach einem der Ansprüche 1 bis 6, **dadurch gekennzeichnet, dass** das Bakterium ausgewählt ist aus *Mycobacterium tuberculosis, Mycobacterium leprae* und *Mycobacterium ulcerans.*

8. Verfahren nach Anspruch 7, **dadurch gekennzeichnet, dass** es sich bei dem Bakterium um ein *Mycobacterium tuberculosis-Bakterium* handelt.

9. Verfahren nach Anspruch 8, **dadurch gekennzeichnet, dass** eine Züchtung in einer mikroaerophilen Inkubationsatmosphäre durchgeführt wird, die einen molaren Sauerstoffanteil von weniger als 20% enthält.

10. Verfahren nach einem der Ansprüche 8 bis 9, **dadurch gekennzeichnet, dass** es sich bei dem Kulturmedium um ein Medium vom Typ Middlebrook handelt.

11. Verfahren nach einem der Ansprüche 8 bis 10, **dadurch gekennzeichnet, dass** das Bakterium *Mycobacterium tuberculosis* in Gegenwart einer antioxidativen Verbindung in einer mikroaerophilen Inkubationsatmosphäre gezüchtet wird, die einen molaren Sauerstoffgehalt von weniger als 5% enthält.

12. Verfahren nach einem der Ansprüche 8 bis 10, **dadurch gekennzeichnet, dass** das Bakterium *Mycobacterium tuberculosis* in Gegenwart einer antioxidativen Verbindung in einer mikroaerophilen Inkubationsatmosphäre gezüchtet wird, bei der es sich um mit bis 5 % C0₂ angereicherte Luft handelt.

13. Verfahren nach Anspruch 10, **dadurch gekennzeichnet, dass** es sich bei dem Kulturmedium um ein festes Kulturmedium auf Basis von Gelose bzw. Agar handelt, vorzugsweise mit 1 bis 2 % Agar.

14. Verfahren nach Anspruch 10, **dadurch gekennzeichnet, dass** das Kulturmedium Wachstumsfaktoren zusätzlicher Mykobakterien umfasst, ausgewählt aus Ölsäure, Rinderalbumin, Rinderalbumin-Fraktion V, Dextrose, Katalase und Glycerin.

15. Verfahren nach einem der Ansprüche 1 bis 12, **dadurch gekennzeichnet, dass** es sich bei dem Medium um ein festes Kulturmedium handelt.

## Claims

1. A process for *in vitro* culture in cell-free culture medium of bacteria whose growth is sensitive to the oxygen content, the optimal growth of said bacteria requiring an incubation atmosphere with a relatively low oxygen content compared with the oxygen content of air, said bacterium being selected from intracellular microaerophilic bacteria of the mycobacteria type, **characterized in that** at least one antioxidant compound, ascorbic acid, is added to said cell-free culture medium at a concentration of at least 100 µg/ml and said bacterium is cultivated in said culture medium in the presence of oxygen.

2. The process as claimed in claim 1, **characterized in that** said culture medium further comprises an antioxidant compound selected from glutathione and sodium hydrosulphide.

3. The process as claimed in claim 1 or 2, **characterized in that** said cell-free medium is selected from:
- an axenic medium consisting of qualitatively and quantitatively defined chemical or biological substances, and
- a medium comprising an extract of ground or lysed multicellular tissue.

4. The process as claimed in one of claims 1 to 3, **characterized in that** said bacterium is cultivated in a said incubation atmosphere comprising a molar proportion of oxygen higher than the maximum tension tolerated in the absence of antioxidant compound for the same level of growth in the same culture period.

5. The process as claimed in one of claims 1 to 4, **characterized in that** said antioxidant compound is used at a concentration of 100µg/ml to 2 mg/ml.

6. The culture process as claimed in one of claims 1 to 5, **characterized in that** said bacterium is a said intracellular microaerophilic bacterium of the mycobacteria type cultivatable in a said cell-free culture medium, under microaerophilic incubation atmosphere with a molar proportion of oxygen of not more than 2.5% in the incubation atmosphere, in the absence of said antioxidant compound, and said bacterium is cultivated in the presence of said antioxidant compound in a said culture medium under an incubation atmosphere comprising a molar proportion of oxygen between 2.5% and 20%.

7. The process as claimed in one of claims 1 to 6, **characterized in that** said bacterium is selected from the bacteria *Mycobacterium tuberculosis, Mycobacterium leprae* and *Mycobacterium ulcerans.*

8. The process as claimed in claim 7, **characterized in that** said bacterium is a *Mycobacterium tuberculosis* bacterium.

9. The process as claimed in claim 8 **characterized in that** a culture is carried out under a microaerophilic incubation atmosphere containing a molar proportion of oxygen of less than 20%.

10. The process as claimed in one of claims 8 to 9 **characterized in that** said culture medium is a Middlebrook type medium.

11. The process as claimed in one of claims 8 to 10 **characterized in that** said *Mycobacterium tuberculosis* bacterium is cultivated in the presence of antioxidant compound under a microaerophilic incubation atmosphere which contains a molar proportion of oxygen of less than 5%.

12. The process as claimed in one of claims 8 to 10 **characterized in that** said *Mycobacterium tuberculosis* bacterium is cultivated in the presence of antioxidant compound under a microaerophilic incubation atmosphere which is 5% CO₂-enriched air.

13. The process as claimed in claim 10 **characterized in that** said culture medium is a solid agar-based culture medium, preferably with 1 to 2% agar.

14. The process as claimed in claim 10 **characterized in that** said culture medium comprises additional mycobacterial growth factors selected from oleic acid, bovine albumin, bovine albumin fraction V, dextrose, catalase and glycerol.

15. The process as claimed in one of claims 1 to 12 **characterized in that** said medium is a solid culture medium.
